# EUROPEAN PATENT APPLICATION

(11) **EP 4 029 928 A1**
(43) Date of publication of application: **20.07.2022**
(21) Application number: 22460001.5
(22) Date of filing: 05.01.2022
(51) Int. Cl.: C12M 1/34

(54) **DEVICE AND METHOD FOR NON-INVASIVE DETECTION AND IDENTIFICATION OF MICROORGANISMS IN SOLID, LIQUID AND GASEOUS SAMPLES**

(30) Priority: 13.01.2021 PL 43665321
(71) Applicant: ML SYSTEM Spólka Akcyjna, 36-062 Zaczernie (PL)
(72) Inventor: KWASNICKI, Pawel, 35-233 Rzeszów (PL)
(74) Representative: Warzybok, Tadeusz

(57) **Abstract**

The subject of the invention is a device and method for the non-invasive detection and identification of microorganisms in solid, liquid and gaseous samples, which is characterized by a rectangular container (1) with a detachably connected cover (6), and a bottom (2) of the container (1) that is detachably connected to a power supply (12) and a controller (13) located opposite the rear end wall (60) of the container (1) and to a CCD detector (18) located opposite the power supply (12) and to a laser light source (23), which is connected by an electric wire (24) to the controller (13), connected by an electric wire (27) to the CCD detector (18), and by an electric wire (28) to the power supply (12), to which 230V/50Hz mains voltage is supplied with an electric wire (29), while the inner surface of the side wall (30) of the container (1) is detachably connected to the connector (32) of the glass light divider (33) perpendicular to it, situated at an acute angle (α) opposite the laser light source (23), and the opposite inner surface of the side wall (30') of the container (1) is also detachably connected to the perpendicular connection (35) of the mirror (36) situated thereto also at an acute angle (α) and parallel to the glass light divider (33), opposite the CCD detector (18) and the front face (37) of the container (1), in which a circular through hole (38) is made against the glass light divider (33) with the first profile connector (40) of the scanning head subassembly (39) embedded therein, while in the opposite rear face (60) of the container (1) there is a through hole (61) with a switch (62) of the controller (13) mounted therein, and next to this switch there is a second through hole (63) with a wire (29) placed in it connected to the power supply (12) and supplying it with the mains voltage from outside the container (1).

## Description

The subject of the invention is a device and method for the non-invasive detection and identification of microorganisms in solid, liquid and gaseous samples of the atmospheric air type, based on the technology of scattering spectroscopy.

Both in the water, soil and air environment as well as on the finished products, there is a large amount of microorganisms that are harmful to human health, such as viruses, bacteria and fungi, and the infection of people with these harmful microorganisms causes various diseases. Therefore, there is a need to prevent infections caused by these microorganisms by detecting them before they enter the human body through droplets, through the ingestion or through direct contact.

Microbes are plant and animal organisms that are subvisible and include, in particular, bacteria, viruses and some fungi. They occur in all environments, colonizing both soil and water as well as living organisms. Therefore, getting to know the physiology and genetics of microorganisms, their interaction with the surrounding environment is very important both for the prevention of infections in humans and animals, and for the protection of the environment and the development of many industries, including the food industry, pharmacy and medicine, as well as for the production of drugs and vitamins.

A method of simultaneous detection of bacteria and fungi in a biological preparation using the Real-Time PCR method/molecular biology/ is known from the Polish patent specification of the invention no. PL228161, in which DNA contained in a sample of biological material is amplified in real-time multiplex PCR in a multiplex system, where the amplification reaction is carried out in two stages with the use of bacterial-specific materials and fungal-specific primers in the first stage, and then the product of the first amplification is used as a matrix in the second stage - amplification with the use of primers and probes distinguishing fungi into a group of mould and yeast-derived fungi and Gram - positive and Gram - negative bacteria.

The Polish patent specification no. PL 218010 provides a method of testing bacterial colonies grown on solid media, which consists in filtering a scaled light beam from a coherent light source in an amplitude filter, polarizing it in a linear polarizer and widening the beam expander with a Iris shutter, its diameter is adjusted and the light beam is focused in the rear focal plane of the plus lens, and the formed convergent spherical light beam illuminates the tested bacterial colony placed on a transparent substrate, placed on a transparent plate mounted in a tripod between the lens and its rear focal plane, then the light beam transmitted and diffracted on the bacterial colony is detected in the detector, and the two-dimensional distribution of the light intensity diffracted on the colony of these bacteria is recorded and analysed in a computer. This computer records and analyses two-dimensional Frensel spectra depending on the morphological structure of the colonies of the analysed bacteria.

The method of characterizing and identifying bacterial colonies with the use of a CCD/ CMOS camera detector, known from the Polish patent specification no. PL232525, consists in focusing the light beam from a coherent light source using optical or optoelectronic phase modulators on the aperture diaphragm, which generates a divergent beam light that illuminates the tested sample in the form of a bacterial colony, placed on a transparent plate mounted in a stand with the possibility of its movement in the X, Y, Z direction. Then, in a detector in the form of a CCD/ CMOS matrix, the result of interference of the transmitted (unbent) non-diffused beam is detected and diffracted bacteria dispersed on the colony in the form of an axial hologram, then the two-dimensional axial digital hologram characteristic for a given species and/or strain of bacteria is recorded and analysed in a computer.

The invention patent specification no. US 8395769 B2 provides a method for assessing the presence of a pathogenic microorganism in a sample that is illuminated so as to produce the first plurality of interacting photons that can be scattered, emitted, reflected and/or absorbed by the sample. The first plurality of interacting photons are assessed to thereby generate a Raman dataset representative of the sample. This Raman dataset is analysed to determine at least one of: the presence of a pathogenic microorganism in said sample and the absence of a pathogenic microorganism in said sample. A Raman dataset may contain at least one of a Raman spectrum and / or a Raman chemical image that is representative for the sample. The analysis may include comparing said Raman data set with at least one reference Raman data set representative for a given sample.

From the invention patent specification no. US 2004219627 A1 a method and apparatus for the automatic detection of bacteria in a sample, especially BACILLINS or COCCUS bacteria, is known, the essence of this method includes: fluorescent staining of bacteria in a sample, detection of information about the size of bacteria in this sample and information about fluorescence that shows the intensity of the fluorescent light emitted by the bacteria, creating a scattergram showing the distribution of bacteria from the size information and the detected fluorescence information, and analysing the distribution of bacteria in the scattergram and determining whether the bacteria in the sample are Bacillus or coccus.

In turn, the apparatus for applying this method includes: a sampling device containing fluorescently stained bacteria, a first detector for detecting information about the size of each bacterium in a sample, a second detector for detecting information about fluorescence expressing the intensity of fluorescent light emitted by each bacterium in mentioned test sample and a control unit configured to create a bacterial scattergram using the fluorescence size information as parameters to analyse the bacterial distribution on the scattergram and to determine whether the bacteria in the mentioned test sample are Bacillus or Coccus bacteria, and the apparatus further includes a flow chamber for the flow of the sample with bacteria and a laser light source for irradiating the sample in the flow chamber, wherein:
- the control unit of this apparatus determines a value representing the state of distribution, determines the maximum direction of variation of the distribution and the slope of the maximum direction of variation, while
- the first detector detects the scattered light obtained from the bacteria and includes a member having pores for passage through the bacteria and two electrodes, wherein the first detector detects the electrical resistance between the first and second electrodes, and the first detector further detects the scattered light emitted by the bacteria in the sample irradiated by laser light source, while
- the second detector detects the fluorescent light emitted by the bacteria in the sample irradiated by this laser light source.

In turn, the CCD (charge-coupled device) matrix known from the publication on the "Wikipedia" website is a system of many photosensitive elements, each of which records and then allows to read an electrical signal proportional to the amount of light falling on it. This matrix, also known as a CCD detector, records the optical image and converts the light signal into an electrical signal (photons into electrons), and it is made on the basis of a transparent silicon wafer. The light falling on this matrix knocks out electrons in individual pixels, the number of which is proportional to the intensity of the light falling on a given pixel. Depending on the intensity of the light and the duration of this exposure of the matrix, different amounts of electric charges accumulate in individual pixels, and their distribution between the pixels reflects the light intensity, which in turn is an "image" of what the camera lens sees.

From the publication on the website "biotechnologia.pl" a compact device known as "Bioarlee Diagnostic Robot" is known, enabling quick and cheap diagnosis of bacteria. A sample taken, for example from human skin, is simply placed in a machine, which, activated by a button on its display, performs processes leading to the identification of the bacteria present in the sample without human intervention. This solution uses a method that uses a laser beam, spectrum registration, an optical system and computer image analysis. The mentioned robot performs a swab on the so-called Petri dish, on which the bacterial colony is multiplied in its incubator, then a laser beam is passed through these bacteria and their image in the form of a spectrum is recorded, using the optical system of this device, and then there is carried out a computer analysis of this image - spectrum using algorithmics and comparison of the spectrum of bacterial colonies with the standard spectrum in a database placed in the cloud.

The purpose of the invention is to develop a simple and compact design of an universal device for the non-invasive detection and identification of microorganisms in the form of viruses, bacteria and fungi in samples taken from solid and liquid materials and in gaseous samples, built on the basis of a high quantum efficiency CCD detector, ensuring the signal to noise equal to SNR<2 and equipped with technical means to expose the tested sample with a coherent beam of electromagnetic wave and collect the spectrum from this sample, scan it and send it to this CCD detector, which will convert this spectrum and present it on a graph in the "X" coordinate system , and "Y" allowing to determine the presence of these microorganisms in the tested sample.

The purpose of the invention is also to develop a method of non-invasive detection and identification of microorganisms such as viruses, bacteria and fungi in samples from solid and liquid materials and in gaseous samples at room temperature and atmospheric pressure using the method of irradiating the tested sample with a coherent beam of electromagnetic wave and collecting the spectrum from the surface of the tested sample and its point irradiation, as well as scanning and detection of this spectrum in the form of a reflected and scattered signal from this sample with the use of a CCD detector.

The device for the non-invasive detection and identification of microorganisms in samples from solid, liquid and gaseous materials according to the invention is characterized in that it has a rectangular container equipped with a detachably connected lid, and the bottom of the container is detachably connected to a power supply and a controller, placed opposite the rear wall of the container and a CCD detector located opposite the power supply and a laser light source, while the laser light source is connected by an electric wire to the controller, connected by further electric wires to the CCD detector and to the power supply, to which the 230V/50Hz mains voltage is connected with the electric wire. In turn, the inner surface of the container sidewall is detachably connected to the perpendicular connector of the glass light divider placed at an acute angle in front of the laser light source, and the opposite inner surface of the second side wall of the container is also detachably connected to the mirror connector placed perpendicularly thereto, also at an acute angle and parallel to the glass light divider, opposite the CCD detector and the front face of the container. In this wall opposite the glass light divider, there is a through round hole with the first profile connector of the scanning head subassembly embedded in it, while in the opposite rear end wall of the container there is a second through hole with a driver switch mounted in it, and next to this switch there is another through hole with a cable placed in it, cable connected to the power supply and supplying with the mains voltage from the outside of the container.

It is preferably when the scanning head subassembly consists of two detachably connected profile sleeve connectors with a glass fiber pin embedded in them, which constitutes the end of one end of a single-mode optical fiber placed in the front part of the profile connector, while the other end of the cable is bent at right angle, placed in an axial hole with the same profile of a two-part cylindrical scanning head with side two half-sleeve offsets located in the axis of symmetry of the hole and equipped with a quartz glass to which the front of the core of the bent end of the single-mode optical fiber is adjacent.

It is also preferably for the two opposing vertical side walls of the power supply, controller and laser light source to adhere and detachably connect the vertical shelves of the three angle members, and their horizontal shelves adhere the bottom of the container and are detachably connected to it, while adjacent to and connected to one vertical wall of the CCD detector is the vertical shelf of the angular element, the horizontal shelf of which is also detachably connected to the bottom of the container.

It is also preferably when the scanning head subassembly is equipped with a multimode optical fiber with a core surrounded by a plastic sheath, covered with a protective sheath, to which two electric wires adhere, surrounded with a protective sheath with a plastic sheath and a protective sheath adhering to it, while the sleeve scanning head is connected to detachably with a sleeve adapter with a lower sleeve shoulder with a smaller diameter than the adapter sleeve, which is equipped with a laser light source on/off switch.

On the other hand, the essence of a non-invasive method of non-invasive detection and identification of microorganisms in samples made of solid and liquid materials or in gaseous samples using a device equipped with a CCD detector, in which the tested sample is irradiated with a laser beam, and then the signal generated from the sample is recorded in the form of an electromagnetic wave and analyses the resulting scatter spectrum characteristic for a given species of these microorganisms in the computer, characterized by the fact that, according to an algorithm implemented in the form of a computer program on a computer, containing a sequence of clearly defined activities necessary to perform specific tasks from the initial state to the final state using a scanning head or sleeve adapter of this head and a single-mode optical fiber for the tested sample made of solid material or the tested water sample, or with the use of a multimode optical fiber for the sample of air exhaled by a person under given tests at room temperature, a spot monochromatic coherent beam of electromagnetic wave ranging from 380 nm to 752 nm is introduced, causing the test sample to be irradiated at the point of contact with the scanning head or its sleeve adapter. Then the resulting spectrum of the tested sample is scanned and the collected signal is transmitted via optical fiber to the detector of this device kept at a temperature of about minus 2°C, ensuring the signal-to-noise ratio for optical signal detection equal to: SNR<2 with detection of this signal in the range of number waveform from 500 cm⁻¹ to 1800 cm⁻¹ and with a spectral resolution of less than 3.5 cm⁻¹, in which the signal transmitted by the scanning head or its sleeve adapter is converted into the spectrum shown on the computer screen in the form of a graph in two coordinates (X, Y), on the abscissa of which the wavenumber is shown in cm⁻¹, and its ordinate axis shows the signal intensity (Arbitrary Unit) and based on the information collected on this graph in the form of peaks, their location and components, the presence of microorganisms in the tested sample and their types are determined, depending on the type of the tested sample, the measurement and determination of the type of microorganisms occurring in it are made in a time ranging from 1 s to 600 s.

Preferably, the transmission of the collected signal from the scanning head to the CCD detector is performed with a single-mode optical fiber, and the signal converted by this CCD detector into a spectrum in the form of a graph is displayed on a computer screen located outside the device for the application of this method.

It is also preferably when the transmission of the collected signal from the scanning head to the CCD detector is performed with a multimode optical fiber, and the signal to spectrum converted by the CCD detector in the form of a graph is presented on a touch screen of a computer installed in the device for the application of this method.

The device according to the invention is characterized by a simple and compact structure, assembled from electronic components and elements available on the market and using typical computers, which significantly simplifies its assembly, while reducing the cost of its production. Moreover, equipping the computer with an algorithm implemented in the form of a computer program, containing a finite sequence of clearly defined activities necessary to perform specific tasks from the initial state to the final state, simplified the operation of this device to the necessary minimum. On the other hand, the use of a measuring system based on dispersed spectroscopy, adapted to the detection of organic samples, including microorganisms such as bacteria, fungi and viruses, does not require special preparation of the sample for testing in the period from several to 600 seconds, and both samples in solid phase as well as samples in liquid and gas phase at room temperature and atmospheric pressure. This measurement can be performed in real conditions and does not require the use of chemical preparation in order to extract and prepare the material for these tests. The significant advantages of both the device and the method of implementation according to the invention also include the fact that the result of the measurement is the spectrum (also called the Raman spectrum) presented on the graph in the ordinate and abscissa (X, Y), on the basis of which the presence of microorganisms can be unequivocally determined, for example bacteria, fungus or virus. The method of detecting microorganisms in tested samples using the device according to the invention does not require the use of chemicals and is completely safe for the environment and the operator performing the measurements. In addition, the device is adapted to continuous operation and can be powered both from the mains or from a battery and is equipped with a monochromatic excitation source with a wavelength preferably of 532 nm and a power of 42 mW at the output and 30 mW on the tested sample, and its irradiation with the monochromatic, coherent beam takes place in a point or in a specific volume thanks to the used scanning head. This head is made in the shape of a cylinder with a diameter not exceeding 25 mm, of copper steel or a polymer based on Teflon, which in turn ensures complete inertia in contact with the material of the tested sample. During the tests of a given sample, the device is designed in such way that the primary beam falls on the sample only through the base of the cylindrical scanning head, and its side wall is completely non-transparent to the beam of an electromagnetic wave in the range from 300 - 752 nm, as a result of which it is possible to collecting the signal only from the tested sample from the places exposed to the primary beam, eliminating at the same time the signal coming from the environment.

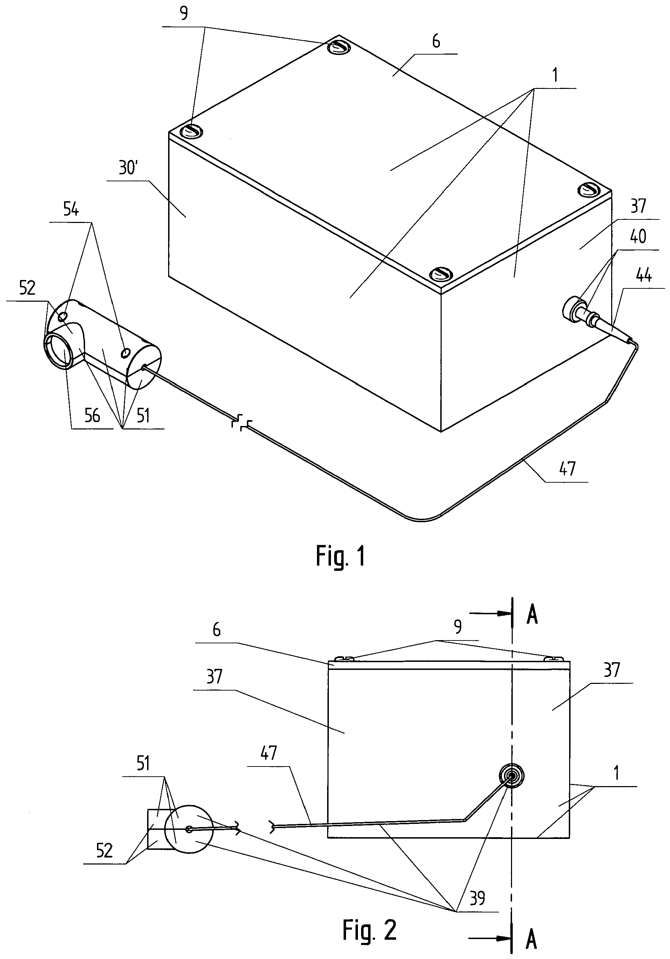

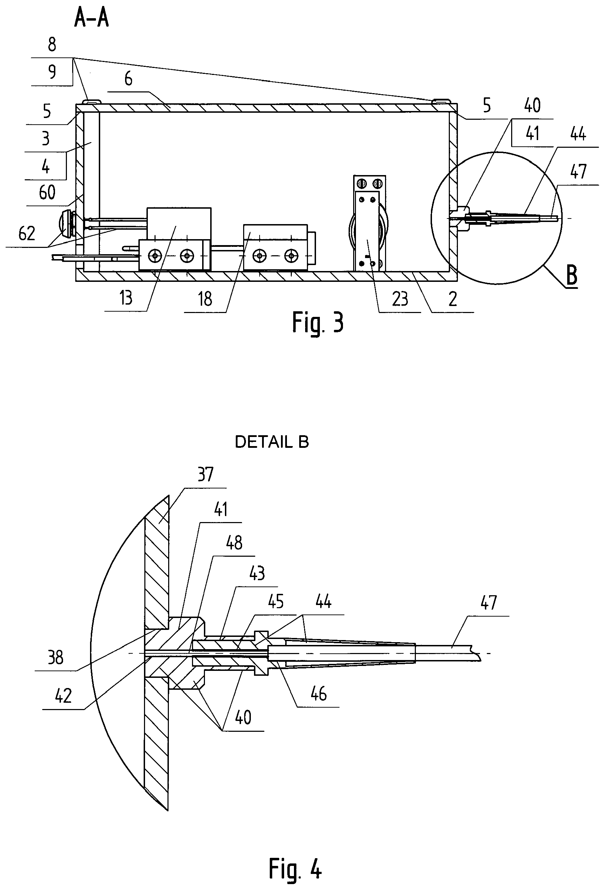

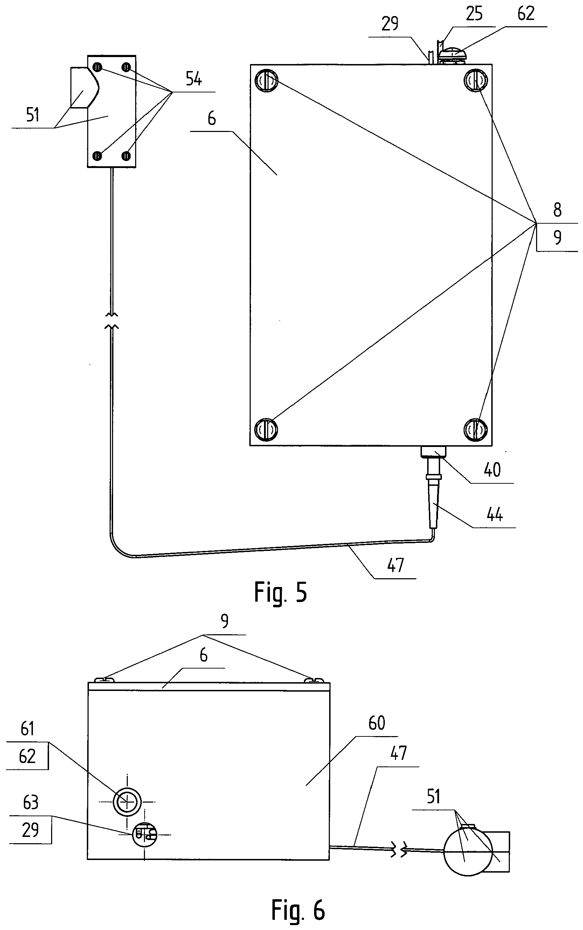

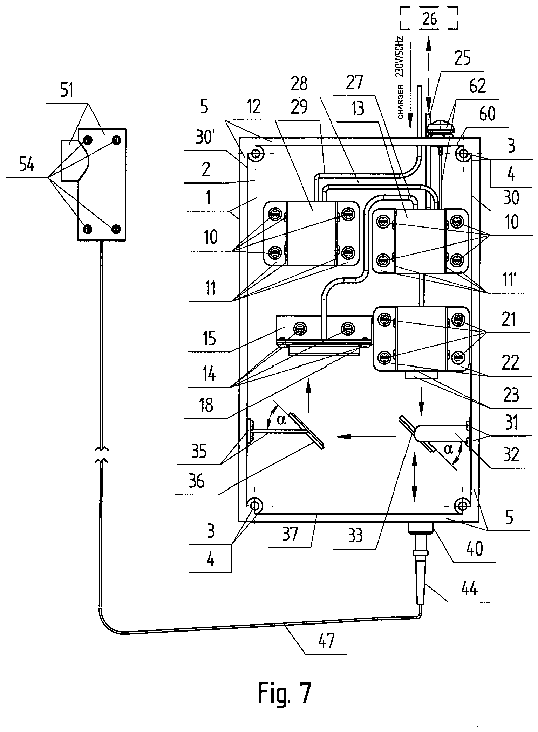

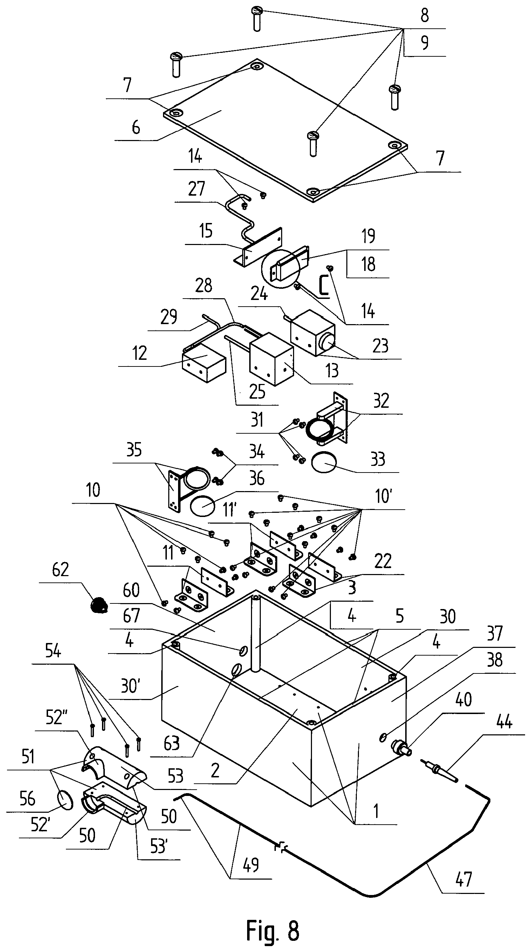

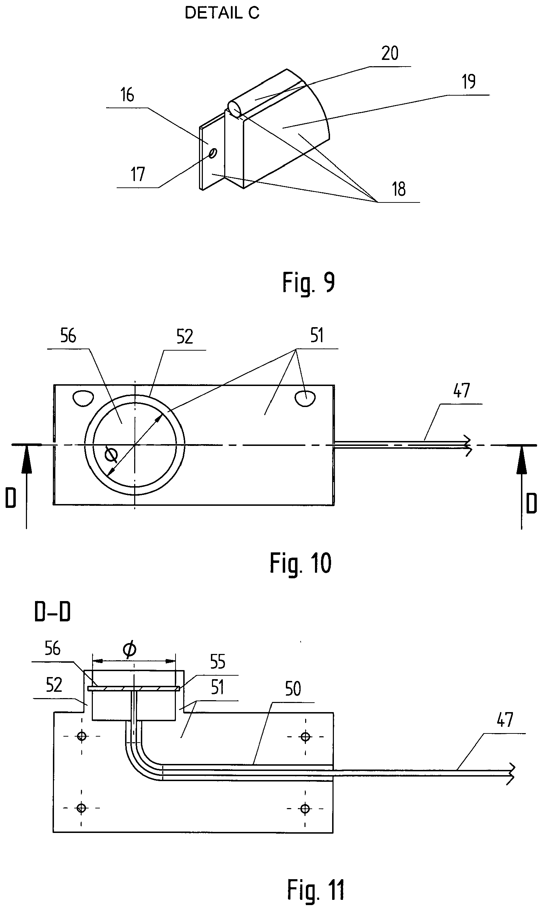

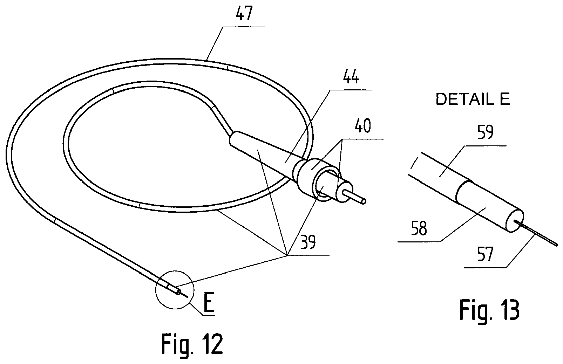

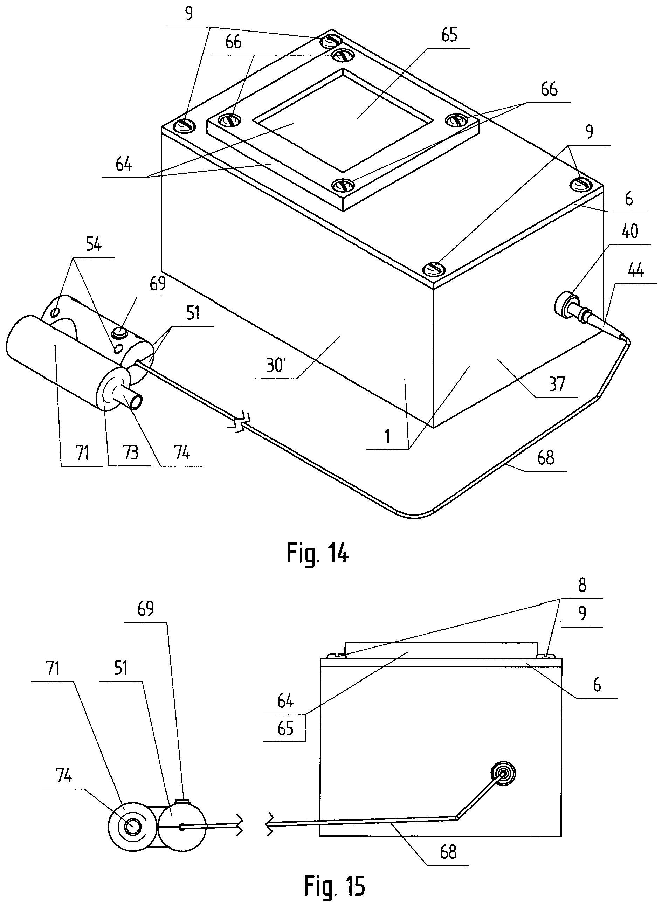

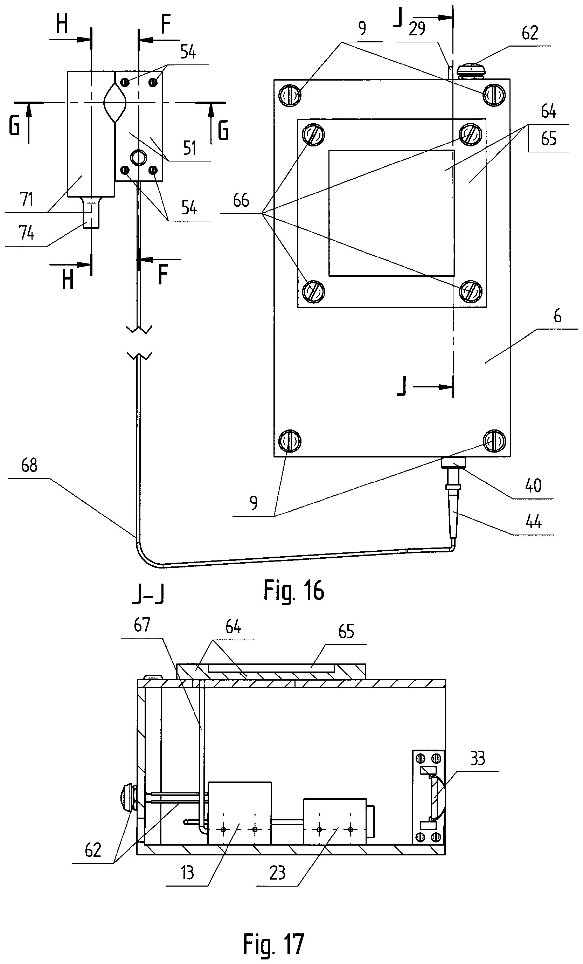

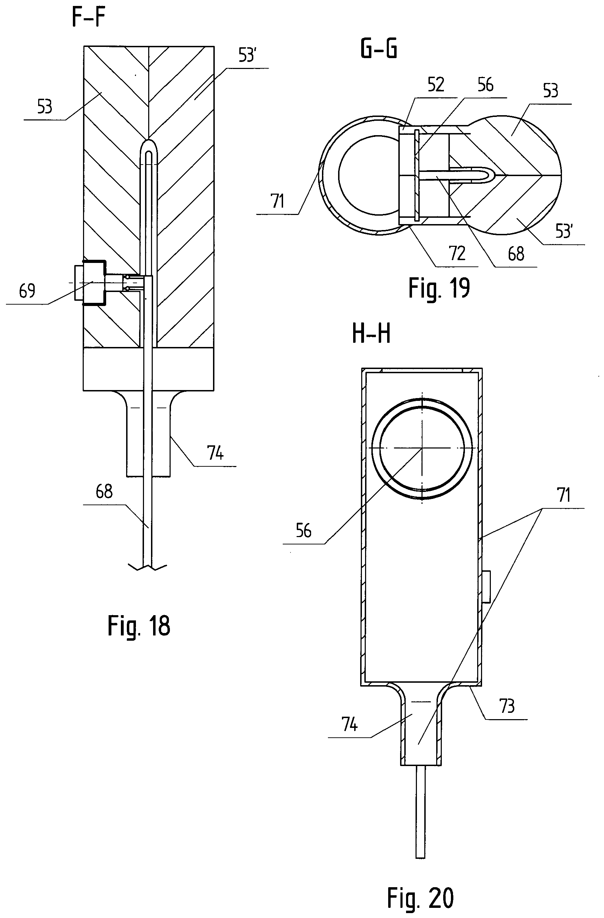

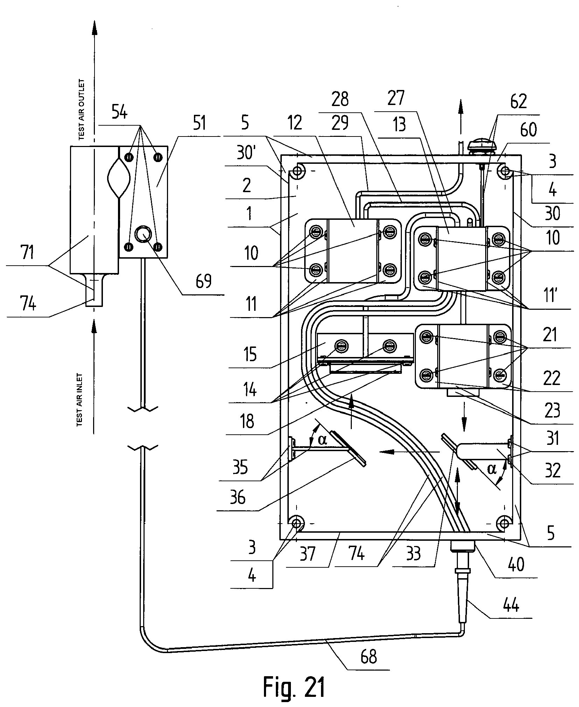

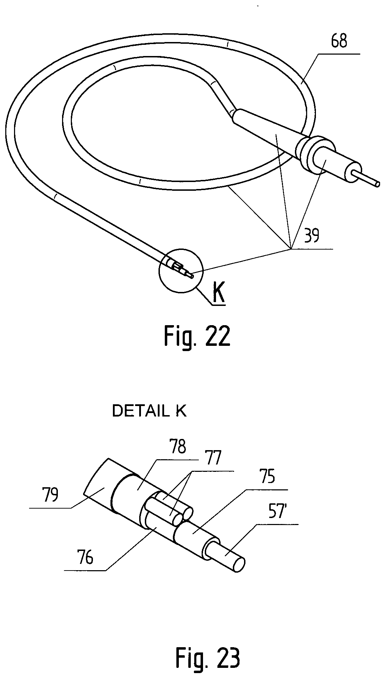

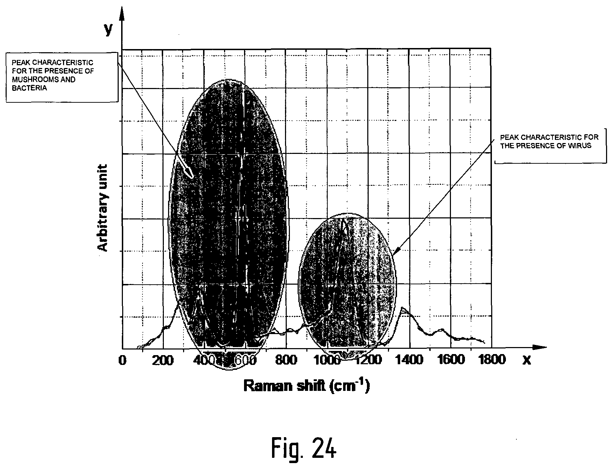

The subject of the invention with regard to the device for the non-invasive detection and identification of microorganisms in samples from solid, liquid and gaseous materials, in two variants of its embodiment, is shown in the figures 1-23, in which figures 1-13 represent the first embodiment variant of this device cooperating with an external computer and equipped with a scanning head sub-assembly, containing a single-mode optical fiber, and figs. 14-26 - the second variant of the device, equipped with a computer with a touch screen and a component of the scanning head connected to the adapter, while the subject of the invention with regard to the method of detecting and identifying microorganisms in samples from solid, liquid and gaseous materials is explained in more detail in four examples of its implementation and in fig. 24, while fig. 1 shows a first variant of the device for detecting and identifying microorganisms in samples from materials solids, liquids and in perspective view in gaseous samples, fig. 2-the same device in the front view from the side of a single-mode fiber extending from it, fig. 3 - the same device in a vertical section along the line A-A, fig. 4-enlarged "B" detail of the sleeve connection of the connector with the pin of a single-mode optical fiber placed in it with the front face of the rectangular container of this device in a vertical section along the line A-A, fig. 5 - the same device in top view, fig. 6 - the same device in a rear view from the side of the power switch mounted in its rear wall, fig. 7 - the same device in top view after removing the cover from its container, fig. 8 - the same device in an exploded state of its components in a perspective view, fig. 9 - an enlarged "C" detail of the CCD detector equipped with a thermocouple in a perspective view, fig. 10 - the device head equipped with a quartz glass contacting one end of a single-mode optical fiber placed inside the head in the front view, fig. 11 - the head of the device itself in a horizontal cross-section along the DD line, fig. 12 - single-mode cable of this device, one end of which is embedded in the connector connected to a second connector embedded in the front face of the device shown in fig. 1, and from the protective outer shell of its other end and the cladding of this optical fiber located therein, the core of this single-mode optical fiber extends outwards, in perspective view, fig. 13 - enlarged "E" detail of the second end of the single-mode optical fiber with the core protruding out from its mantle, placed in the cylindrical scanning head and its sleeve offset, in a perspective view, fig. 14-shows the second embodiment of the device for detecting and identifying microorganisms in samples from solid materials, in a perspective view, fig. 15-the same device in a front view from the side of a multimode fiber extending from it, fig. 16 - the same device in a top view, fig. 17 - a fragment of a container with its cover and a computer connected to it in a vertical section along the JJ line, fig. 18. - the head of the same device in a vertical section along the FF line, fig. 19-the head connected to the adapter of the scanning head component in a vertical section along the GG line, fig. 20 - the adapter of the scanning head component in a vertical section along the HH line, fig. 21 - the device itself in a top view after removing the cover from its container, fig. 22 - a multimode cable of this device, one end of which is embedded in a first profile connector connected to a second profile connector embedded in the front face of the device shown in fig. 14, and fig. 23, an enlarged "K" detail of the other end of the multimode optical fiber with the core extending out from its mantle, located in the cylindrical scanning head and its sleeve offset, in a perspective view.

### Example 1

The device for non-invasive detection and identification of microorganisms in solid, liquid and gaseous samples according to the first version of its embodiment shown in figs. 1-13 has a rectangular container 1 with a rectangular bottom 2, having in its corners a 3 cylindrical-sleeve offsets with the inner thread 4 in their upper sleeve parts, to which and to the upper horizontal faces 5 of the side walls of this container, a plate cover 6 is adjacent with through round holes 7 made in their corners, in which there are embedded screws 8, screwed into the threaded upper ends of the sleeve parts of the offsets 3 so that the heads 9 of these screws are adjacent to the upper surface of the cover 6, sealing it tightly with the upper faces 5 of the side walls of the container 1. Two pairs of angle elements 11 and 11' are attached to the bottom 2 of the container 1 in the rear part of the device by means of screws 10, and the vertical shelves of angle elements 11 are fastened with screws 10 and attached to the side walls of the power supply 12 placed between them, while both vertical shelves of the angular elements 11' screwed by means of screws 10' are attached to the side walls of the controller 13 placed between them. Moreover, to the bottom 2 of the container 1 in front of and opposite the power supply 12 and the controller 13, one angle element 15 is fastened by means of screws 14, the vertical shelf of which, , is also attached to the side shoulders 16 by means of screws 14 with holes 17 of the CCD 18 detector threaded therein, equipped with a photosensitive matrix 19 transforming the light signal into an electrical signal and in a thermocouple 20 maintaining the matrix at a temperature of minus 2°C, next to this detector and opposite the controller 13, to the bottom 2 by means of screws 21 are attached two opposite angle elements 22, the vertical shelves of which, also by means of screws 21, are attached to the side walls of the laser light source 23 placed between them, connected via an electric wire 24 to the controller 13, which is connected with an electric wire 25 to an external computer 26 with an algorithm implemented in the form of a computer program containing a finite sequence of clearly defined steps necessary to perform specific tasks from the initial state to the final state, the CCD detector 18, by means of an electric wire 27, is also connected to the controller 13, which by an electric wire 28 is connected to a power supply 12 connected by an electric wire 29 to an external voltage source of 230V/50 Hz. In turn, to one inner side surface 30 of the container 1 by means of screws 31 is attached the connector 32 of the circular glass light divider 33 located at an angle α = 45° and opposite the laser light source 23, and opposite the light divider 33 to the other opposite inner side surface of the side wall 30', with the help of screws 34, there is attached the connector 35 of the mirror 36 arranged parallel to the light divider 33 and at an angle α = 45 °, the connectors 32 and 35 situated perpendicular to the inner surface of the side walls 30 and 30' of the container 1. Moreover, in the front wall 37 of the container 1, a circular through hole 38 is provided opposite the laser light source 23, in which a scanning head subassembly 39 is mounted, consisting of a profile connector 40 with an annular flange 41 and an internal two-stage axial circular hole 42 and 43 with a profile mounted therein a sleeve connector 44 also with a two-stage diameter 45 and 46 with one end of a single-mode optical fiber 47 terminated with a glass fiber pin 48, the front end of which is also embedded in the opening 42 of the profile connector 40. On the other hand, the second end 49 of the right-angled single-mode optical fiber is placed in an axial opening 50 of the same profile, made in a two-part, a cylindrical scanning head 51 made of stainless steel with a diameter of 25 mm with two lateral half-sleeve offsets 52' and 52", constituting a sleeve 52, both half-cylindrical parts 53 and 53' of which and both half-sleeves 52' and 52" are connected to each other by means of screws 54, forming one monolith of this head, and inside the front part of the so formed and offset sleeve 52 a round channel 55 with a quartz glass 56 is made with an active diameter of ø = 20 mm inside it, while the single-mode optical fiber 47 has a core 57 in contact with the glass 56, which is surrounded by a plastic sheath 58, covered with a protective coating 59. Moreover, in the rear wall 60 of the container 1 opposite of the controller 13, a through hole 61 is made with a switch 62 of electric power supply to the controller mounted therein 13, and next to this switch, a second through hole 63 is provided in this rear face 60 with a wire 29 disposed therein for supplying 230V/50Hz mains voltage to the power supply 12 of this device.
At the same time, with a view to simplifying and reducing the cost of this device, in the design solution, the profile connector 40 was replaced with a typical St/Pc connector, and the profile connector 44 was replaced with a simple connector.

The principle of operation of this device is based on the fact that after connecting the power supply cable 12 to the external 230V/50Hz AC main, the switch 62 turns on the device, and its controller 13, via an electric cable 25, connects to the computer 26 and its parameters are set, then, by means of this computer, the laser light source 23 is switched on, which passes through the light splitter 33 and is directed to pin 48, the first profile connector 40, and then to one end of the single-mode optical fiber 47 embedded therein, through which this light is transmitted to the scanning head 51 and to its quartz glass 56, through which it passes to a suitable test sample placed on a substrate (not shown). Then the sample is irradiated, and the light reflected from it and scattered through the scanning head 51 and the single-mode optical fiber 47 placed therein returns through the profile connectors 44 and 40 of the scanning head sub-assembly 39 to the light splitter 33, located at an angle α = 45°, where the part of the light is reflected and directed to a mirror 36 also at an angle of 45°, from which it is reflected and at an angle of 90° hits the photosensitive matrix 19 of the CCD detector 18, which converts this light signal into an electrical signal sent to the controller 13 and to a computer 26 that processes these signals into the reflected light spectrum, which is analysed to detect and identify the type of microorganisms (fungi, bacteria or viruses) in the tested sample, which is a solid or liquid material, with the spectrum in the coordinate system Raman Shift (cm⁻¹) Arbitrary Unit as shown in fig. 24 displayed on the screen of the computer.

### Example 2

The device for the non-invasive detection and identification of microorganisms in samples from solid, liquid and gaseous materials according to the second embodiment, has a structure similar to that described in the first embodiment of the device according to the first embodiment, and the difference between the two types is only that in device according to the second embodiment:
- the lid 6 of the container 1 is equipped with a computer 64 with a touch screen 65 and an algorithm implemented in the form of a computer program, containing a finite sequence of clearly defined activities necessary to perform specific tasks, from the initial state to the final state, the computer being connected by means of screws 66 with the cover 6, and connected by a wire 67 to the controller 13 of this device, while
- the sub-assembly of the scanning head 39 is equipped with a multimode optical fiber 68, and its sleeve scanning head 51 made of a Teflon-based polymer with a diameter of 22 mm, has an on/off switch 69 of a laser light source 23, and its two-piece monolithic sleeve offsets 52' and 52" constituting a sleeve 52, with a quartz glass 56 embedded in it and the bent end of the core 57' of a multimode optical fiber 68 adjacent to it, is connected to a sleeve adapter 71, in the round wall of which a circular hole 72 is made with a diameter adapted to the outer diameter of the sleeve 52, in which it is pressed in, which allows for easy disassembly in the event of the need to test gas samples.

Moreover, this sleeve adapter 71 has a sleeve shoulder 74 on its lower face 73, which acts as a mouthpiece for the blown tested air. In addition, the core 57' of the multimode optical fiber 68 is surrounded by a plastic jacket 75, covered with a protective sheath 76, to which two electric wires 77 adhere, surrounded with this sheath by a plastic jacket 78 and a protective sheath 79 adjacent to it, and both electric wires 77 are connected with switch 69 for laser light source 23.

In case of testing samples from solid and liquid materials the principle of operation of this second embodiment of the device according to the invention is similar to the principle of operation of the above-described first embodiment of this device, and the difference between the operating principles of both types of devices is that before testing a gas sample the sleeve-shaped scanning head 51 should be press-fitted with the sleeve adapter 71. The tested air is then supplied through the sleeve shoulder 74 of the adapter 71, for example by a person who blows his exhaled air through this lower sleeve which travels the entire length of the adapter 71, at the same time having contact with the quartz glass 56 of the scanning head 51, from which the reflected light from this glass is transmitted by the multimode optical fiber 68 to the computer 64 in the manner described in the principle of operation of the first variant of this device.

### Example 3

A 5 x 5 cm sample was cut from a sheet of paper intended for packaging food products and placed on a glass substrate, then on the surface of this paper sample at room temperature there was placed the face of the sleeve offset 52 with a quartz glass 56 of scanning head 51 with a diameter of ø = 20, connected by a single-mode fiber 47 with a diameter of 8 µm to a CCD detector 18 (charge-coupled device) maintained at a temperature of minus 2°C by a thermocouple ensuring a signal-to-noise ratio of SNR<2, wherein switching on the controller 13 connected to the computer 26 equipped with an algorithm recording the performed activities of this detector by means of this optical fiber, a point monochromatic coherent beam of electromagnetic wave of 300 nm was introduced into this paper sample, causing the tested sample to be irradiated at the point of its contact with the scanning head and collecting spectrum from this test area with a diameter of 20 mm. Then the obtained signal in the form of this spectrum reflected from the tested paper sample was collected by this scanning head 51 and fed with this fiber to the CCD 18 detector with high quantum efficiency ensuring the possibility of detecting this signal in the wavenumber range from 500 cm⁻¹ to 1800 cm⁻¹ with spectral resolution below 3.5 cm⁻¹. The collected signal was converted by this detector into the characteristic Raman spectrum, shown on the computer screen 26 in the form of a graph in the coordinate system (X, Y), on the ordinate axis (Y) the Arbitrary Unit of which, dimensionless signal intensity was presented, and the abscissa (X ) Raman Shift wavenumber measured in cm⁻¹ (as shown in fig. 24), where the time of this measurement was 10 s, as a result of which the signal collected in the form of their location peaks and components allowed to establish that in this sample the presence of fungi and bacteria as well as the presence of the virus were detected, by presenting them in the form of peaks characteristic for them.

### Example 4

From the so-called "swab" of human tissue from a living human, there was taken a swab - a sample, which was also placed on a glass substrate, and then to this sample - swab at room temperature, there was placed the front of the sleeve insert 52 with a quartz slide 56 with a diameter of ø = 20 mm of scanning head 51 connected by a multimode fiber 50 µm diameter with a CCD detector 18 (charge-coupled device) maintained at a temperature of minus 2°C by a thermocouple 20 ensuring a signal-to-noise ratio of SNR<2, and after switching on the controller 13 connected to the computer 26 equipped with an algorithm recording the performed activities of this detector by means of optical fiber 47, a point monochromatic coherent beam of electromagnetic wave of 752 nm was introduced into this sample, causing the tested sample to be irradiated at the point of contact with this scanning head and the spectrum was collected from this tested area with a diameter of 1 mm. Then the obtained signal in the form of this spectrum reflected from the tested sample of human tissue was collected by this scanning head and fed with this optical fiber to the CCD 18 detector with high quantum efficiency ensuring the possibility of detecting this signal in the wavelength range from 500 cm⁻¹-1800 cm⁻¹ with spectral resolution of 3.45cm⁻¹. The collected signal was converted by this detector into the characteristic Raman spectrum shown in the diagram (fig. 24) described in the third example, the time of this measurement was 600 s, as a result of which the collected signal in the form of their location peaks and components allowed to determine, that in this sample of human tissue, the presence of similar fungi, bacteria and viruses was detected.

### Example 5

200 ml of clean water was poured into a typical glass, and then, at room temperature and atmospheric pressure, a quartz slide 56 with a diameter of ø = 20 mm of the sleeve shoulder 52 of the scanning head 51 was immersed in this water to a depth of 1 mm, so that its surface immersed in this water was situated parallel to the upper surface of this water. The head was connected by means of a planar single-mode optical fiber 47 with a diameter of 8 µm to a CCD detector 18 (charge-coupled device) maintained at a temperature of minus 2°C by means of a thermocouple 20 ensuring that the signal-to-noise ratio of SNR<2 was maintained, wherein controller 13 connected to a computer 26 equipped with an algorithm recording the activities of this detector using this optical fiber, a point monochromatic coherent beam of electromagnetic wave of 380 nm was introduced into this water sample, causing the tested water sample to be irradiated at the point of contact with the lower surface of the immersed head in the test water and collecting the spectrum from this test water sample with a diameter of 10 mm. Then, the obtained signal in the form of this spectrum reflected from the tested water sample was collected by this scanning head and fed by this optical fiber to the CCD 18 detector with high quantum efficiency ensuring the possibility of detecting this signal in the wavenumber range from 500 cm⁻¹ - 1800 cm⁻¹ with a spectral resolution of 2.0 cm⁻¹. The collected signal was converted by this detector into the characteristic "Raman" spectrum shown in the graph (fig. 24) described in the third example, the time of this measurement was 300 s, as a result of which this collected signal in the form of their location peaks and components allowed for establishing that in this water sample the existence of fungi, bacteria and viruses was also detected, presenting them in the form of their characteristic peaks.

### Example 6

A scanning head 51 was used, connected at one end by a multimode fiber 68 to a CCD detector 18, analogously to that described in the first example, while a sleeve shoulder 52 with a quartz glass 56 of this head was connected directly to the adapter 71 (as shown in figs. 14-21), then also at room temperature and at normal atmospheric air pressure, a person subjected to tests aimed at detecting and identifying microorganisms in his body, inhaled the air exhaled by him directly into the interior of the sleeve mouthpiece 72, which is the lower face of this adapter. The flowing stream of blown air under a pressure of 10-100 mbar was irradiated with an electromagnetic wave beam of 532 nm and power of 42 mW at the output and 30 mW on the tested sample, and it allowed to collect the spectrum from this tested sample of blown air. Then, the obtained signal in the form of this spectrum reflected from the tested water sample was collected by the scanning head 51 and sent by means of a multimode fiber 68 to the CCD 18 detector with high quantum efficiency ensuring the possibility of detecting this signal in the wavenumber range from 500 cm⁻¹ to 1800 cm⁻¹ with a spectral resolution of 2.0 cm⁻¹. The collected signal was converted by this detector into the characteristic "Raman" spectrum presented in the graph (fig. 24) described in the third example, the time of this measurement being 20 s, as a result of which this collected signal in the form of their location peaks and components allowed to determine that the presence of similar fungi and bacteria was detected in this water sample.

## Claims

1. Device for the non-invasive detection and identification of microorganisms in solid, liquid and gaseous samples, equipped with a CCD detector and a laser light source, **characterized by** a rectangular container (1) with a detachably connected cover (6), and the bottom (2) of the container (1) that is detachably connected to a power supply (12) and a controller (13) facing the rear end wall (60) of the container (1) and to a CCD detector (18) located opposite the power supply (12) and to the controller (13) and a laser light source (23), which is connected by an electric wire (24) to the controller (13), connected by an electric wire (27) to the CCD detector (18), and by an electric wire (28) to the power supply (12), to which 230V/50Hz mains voltage is supplied with an electric wire (29), while the inner surface of the side wall (30) of the container (1) is detachably connected to the connector (32) of the glass light divider (33) perpendicular to it, situated at an acute angle (α) opposite the laser light source (23), and the opposite inner surface of the side wall (30') of the container (1) is also detachably connected to the perpendicular connection (35) of the mirror (36) situated thereto also at an acute angle (α) and parallel to the glass light divider (33), opposite the CCD detector (18) and the front face (37) of the container (1), in which a circular through hole (38) is made against the glass light divider (33) with the first profile connector (40) of the scanning head subassembly (39) embedded therein, while in the opposite rear face (60) of the container (1) there is a through hole (61) with a switch (62) of the controller (13) mounted therein, and next to this switch there is a second through hole (63) with a wire (29) placed in it connected to the power supply (12) and supplying it with the mains voltage from outside the container (1).

2. The device according to claim 1, **characterized in that** the scanning head subassembly (39) consists of two detachably connected profile sleeve connectors (40 and 44) with a glass fiber pin (48) embedded in them, constituting the end of one end of a single-mode optical fiber (47) placed in front of the profile connector (40), while the other end (49) of the optical fiber, bent at a right angle, is placed in an axial opening (50) with the same profile of the two-part cylindrical scanning head (51) with side two half-sleeve offsets (52', 52"), positioned in the symmetry axis of the hole (50) and equipped with a quartz glass (56) against which the front of the core (57) of the bent end (49) of the single-mode optical fiber (47) is adjacent.

3. Device according to claim 1, **characterized in that** the vertical shelves of the angle elements (11, 11' and 22) are adjacent and detachably connected to the two opposite vertical side walls of the power supply (12), the controller (13) and the laser light source (23) and their horizontal shelves adhere to the bottom (2) of the container (1) and are detachably connected to it, while one vertical wall of the CCD detector (18) is adjacent and connected to the vertical shelf of the angular element (15), the horizontal shelf of which is also detachably connected to the bottom (2) of the container (1).

4. Device according to claim 1 or 2 or 3, **characterized in that** the scanning head subassembly (39) is equipped with a multimode fiber (68) with a core (57') surrounded by a plastic jacket (75), covered with a protective sheath (76), to which two electric wires 77 adjoin, surrounded with a protective sheath (76) with a plastic jacket (78) and an adjacent protective sheath (79), while the sleeve scanning head (51) is detachably connected with the sleeve adapter (71) with the lower shoulder sleeve (74) with a diameter smaller than the adapter sleeve (71), which is equipped with an on/off switch (69) of the laser light source (23).

5. A method of non-invasive detection and identification of microorganisms in samples from solid and liquid materials or in gaseous samples using a device equipped with a CCD detector, in which the tested sample is subjected to irradiation with a laser beam, and then the signal generated from the sample is recorded in the form of an electromagnetic wave and the resulting scatter spectrum characteristic for a given species of these microorganisms is analysed on a computer, **characterized by** the fact that according to the algorithm implemented in the form of a computer program in a computer (26, 64) containing a sequence of clearly defined activities necessary to perform specific tasks from the initial state to the final state using the scanning head (51 ) or a sleeve adapter (71) of this head and a single-mode optical fiber (47) for a test sample made of solid material or a test water sample, or by means of a multi-mode optical fiber (68) for a sample of air exhaled by a test subject at room temperature, a point monochromatic coherent electromagnetic wave beam ranging from 380 nm to 752 nm is introduced, causing the test sample to be irradiated at the point of contact with the scanning head (51) or its sleeve adapter (71), then the resulting spectrum of the tested sample is scanned and the collected signal is transmitted via optical fiber (47 or 68) to the detector (18) of this device kept at a temperature of about minus 2°C, ensuring the signal-to-noise ratio for the detection of the optical signal is: SNR<2 with the detection of this signal in the wavenumber range from 500 cm⁻¹ to 1800 cm⁻¹ and with a spectral resolution of less than 3.5 cm⁻¹, in which the signal transmitted by the scanning head (51) or its sleeve adapter (71) is converted into a spectrum shown on the computer screen (26 or 64) in the form of a graph in the two-coordinate system (X, Y), on the abscissa axis of which is presented the number of waves measured in cm⁻¹, and its ordinate axis shows the signal intensity (arbitrary unit) and based on the information collected in this graph in the form of peaks, their location and components, the presence of microorganisms and their types in the tested sample are determined.

6. The method according to claim 5, **characterized in that,** depending on the type of the tested sample, the measurement and determination of the type of presence of microorganisms in it are performed in a time ranging from 1 s to 600 s.

7. The method according claim 5 or 6, **characterized in that** the transmission of the collected signal from the scanning head (51) to the CCD detector (18) is performed by a single-mode optical fiber (47), and the signal converted by the CCD detector (18) to the spectrum is presented in the form of a plot in the coordinate system on a computer screen (26) located outside the apparatus for implementing the method.

8. The method according to claim 5 or 6 or 7, **characterized in that** the transmission of the collected signal from the scanning head (51) to the CCD detector (18) is performed by a multimode optical fiber (68), and the signal converted by the CCD detector (18) into a spectrum in the form of a plot in the coordinate system is shown on a touch screen (65) of a computer (64) mounted on an apparatus for implementing of the method.
